# EUROPEAN PATENT SPECIFICATION

(11) **EP 2 203 422 B1**
(45) Date of publication and mention of the grant of the patent: **22.06.2011**
(21) Application number: 08842890.9
(22) Date of filing: 23.10.2008
(51) Int. Cl.: C07D 207/14, A61K 31/40, A61P 25/00

(54) **PHENYLPYRROLIDINE COMPOUNDS**
PHENYLPYRROLIDINVERBINDUNGEN
COMPOSÉS PHÉNYLPYRROLIDINIQUES

(30) Priority: 25.10.2007 ES 200702800
(43) Date of publication of application: 07.07.2010
(73) Proprietor: Ferrer Internacional, S.A., 08028 Barcelona (ES)
(72) Inventor: FALCÓ, José, E-08004 Barcelona (ES); PALOMER, Albert, E-08014 Barcelona (ES); GUGLIETTA, Antonio, E-08750 Molins De Rei (ES)
(74) Representative: Reitstötter - Kinzebach
(86) International application number: PCT/EP2008/064390
(87) International publication number: WO 2009/053441

(56) References cited:
- WO-A-01/02392
- DIETER STEINHILBER ET AL: "Melatonin receptor ligands" EXPERT OPINION ON THERAPEUTIC PATENTS, INFORMA HEALTHCARE, GB, vol. 9, no. 3, 1 January 1999 (1999-01-01), pages 281-290, XP002444600 ISSN: 1354-3776

## Description

### Field of the art

The present invention belongs to the field of compounds with activity on melatonin receptors, especially phenylpyrrolidines, and more specifically acylated 1-(3-alkoxy-phenyl)-pyrrolidin-3-yl-amines.

### State of the art

Insomnia is the most common sleep disorder and affects 20-40% of adults, with a frequency that increases with age. Insomnia has many causes. One of these is the interruption of the normal wakefulness-sleep cycle. This dyssynchrony may result in pathological changes. A potential therapeutic treatment that allows correcting said effect consists in re-synchronising the wakefulness-sleep cycle by modulating the melatoninergic system (Li-Qiang Sun, Bioorganic & Medicinal Chemistry Letters 2005, 15, 1345-49).

Melatonin is a hormone segregated by the pineal gland that is responsible for information on the light-dark cycles, for controlling the circadian rhythm in mammals and for modulating retinal physiology. Melatonin synthesis and its nightly secretion are controlled by the suprachiasmatic nucleus and synchronised by environmental light (Osamu Uchikawa et al., J. Med. Chem. 2002, 45, 4222-39; Pandi-Perumal et al., Nature Clinical Practice 2007, 3 (4), 221-228).

Melatonin secretion in humans occurs simultaneously to sleep at night, and the increase in melatonin levels is correlated with the increase in the desire to sleep during the evening.

In humans, the clinical applications of melatonin range from treatment of the delayed sleep phase syndrome to jet lag treatment, including treatment applied to night shift workers and as a hypnotic treatment.

Melatonin receptors have been classified as MT1, MT2 and MT3 based on pharmacological profiles. The MT1 receptor is located in the hypothalamus central nervous system, whereas the MT2 receptor is distributed throughout the central nervous system and the retina. The presence of MT1 and MT2 receptors has been described at the peripheral level. The MT1 and MT2 receptors are involved in a large amount of pathologies, the most representative of these being depression, stress, sleep disorders, anxiety, seasonal affective disorders, cardiovascular pathologies, digestive system pathologies, insomnia or fatigue due to jet lag, schizophrenia, panic attacks, melancholia, appetite disorders, obesity, insomnia, psychotic diseases, epilepsy, diabetes, Parkinson's disease, senile dementia, disorders associated to normal or pathological aging, migraine, memory loss, Alzheimer's disease and brain circulation disorders. The MT3 receptor has been recently characterised as the homologue of the quinone reductase-2 (QR2) enzyme. MT1 and MT2 are G protein-coupled receptors (GPCR), the stimulation of which by an agonist leads to a reduction in adenylate cyclase activity and the resulting reduction in intracellular cAMP.

Bioorganic & Medicinal Chemistry letters (2003) 4381-4384 discloses (S)-N-[1-(3)-methoxy-phenyl)-pyrrolidine-3-yl]-butyramide which has good affinity for MT1 and MT2 receptors, but the same vasoconstrictive activity as melatonin itself in an assay conducted with rat caudal arteries.

Patents US 4600723 and US 4665086 advocate the use of melatonin to minimise alterations of the circadian rhythms that occur due to changes in work shifts from days to nights or from passing quickly through several time zones in an airplane (jet lag). Several families of compounds with melatoninergic activity had been described in patent documents EP 848699B1, US 5276051, US 5308866, US 5633276, US 5708005, US 6034239 (ramelteon), US 6143789, US 6310074, US 6583319, US 6737431, US 6908931, US 7235550, WO 8901472, WO 01/02392, WO 01/02392 and WO 2005062992.

Patent application WO 9608466 describes indane compounds as ligands to melatonin receptors belonging to formula: 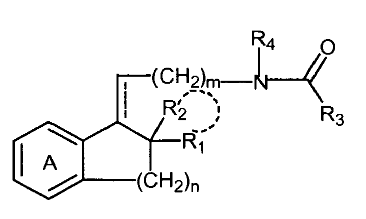 wherein substituents R₁, R₂, R₃ and R₄ and variables A, m and n have the meanings described therein.

Ramelteon, N-[2-[(8S)-1,6,7,8-tetrahydro-2H-indeno[5,4-b]furan-8-yl)ethyl]propionamide, is the first melatonin agonist introduced in therapy. It is indicated in insomnia and its mechanism of action is based on the agonism of the MT1 and MT2 receptors.

Ramelteon is a non-selective compound against MT1 and MT2, and selective against other receptors at the central and peripheral level. Its Ki is 0.014 nM for MT1 and 0.045 nM for MT2. It has resorption, but experiences an important first-pass metabolic effect. It is biotransformed into four metabolites, one of these being M-II, active and with an important distribution volume. Ramelteon clearance is 88%.

The research of new melatonin agonists that may be useful in the treatment of insomnia responds to a fundamental health need, and therefore justifies continued research for compounds with improved properties.

Therefore, the present invention is aimed at new acylated 1-(3-alkoxyphenyl)-pyrrolidin-3-yl-amines that are active against melatonin receptors, especially MT1 and MT2 receptors. As a result, the compounds of the present invention are useful in the treatment and prevention of all those diseases that are mediated by MT1 and MT2 receptors. Some non-limiting examples of melatoninergic disorders are depression, stress, sleep disorders, anxiety, seasonal affective disorders, cardiovascular pathologies, digestive system pathologies, insomnia or fatigue due to jet lag, schizophrenia, panic attacks, melancholia, appetite disorders, obesity, insomnia, psychotic diseases, epilepsy, diabetes, Parkinson's disease, senile dementia, disorders associated to normal or pathological aging, migraine, memory loss, Alzheimer's disease and brain circulation disorders.

### Detailed description of the invention

The present invention relates to phenylpyrrolidine compounds of general formula I 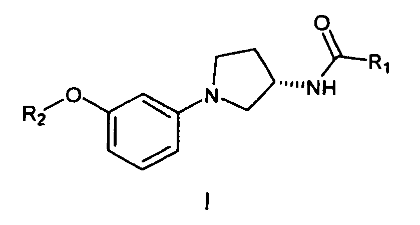 selected from the group consisting of
1) (S)-N-[1-(3-methoxy-phenyl)-pyrrolidin-3-yl]-2,2-dimethyl-propionamide;
2) (S)-N-[1-(3-methoxy-phenyl)-pyrrolidin-3-yl]-isobutyramide;
3) (S)-N-[1-(3-methoxy-phenyl)-pyrrolidin-3-yl]-propionamide;
4) (S)-N-[1-(3-methoxy-phenyl)-pyrrolidin-3-yl]-acetamide;
5) (S)-N-[1-(3-methoxy-phenyl)-pyrrolidin-3-yl]-cyclopropanecarboxamide;
6)(S)-N-[1-(3-methoxy-phenyl)-pyrrolidin-3-yl]-pentanamide;
7) (S)-N-[1-(3-methoxy-phenyl)-pyrrolidin-3-yl]-3-methyl-butyramide;
8) Methyl (S)-[1-(3-methoxy-phenyl)-pyrrolidin-3-yl]-carbamate;
9) Ethyl (S)-[1-(3-methoxy-phenyl)-pyrrolidin-3-yl]-carbamate;
10) (S)-2,2,2-trifluoro-N-[1-(3-methoxy-phenyl)-pyrrolidin-3-yl]-acetamide;
11) (S)-2-fluoro-N-[1-(3-methoxy-phenyl)-pyrrolidin-3-yl]-propionamide; and
12) (S)-3-[1-(3-methoxy-phenyl)-pyrrolidin-3-yl]-1-ethylurea;
and pharmaceutically acceptable salts and hydrates thereof.

Pharmaceutically acceptable salts are those that may be administered to a patient, such as a mammal (e.g. salts with acceptable safety in mammals for a given dosing regimen). Such salts may be obtained from pharmaceutically acceptable inorganic and organic bases and from pharmaceutically acceptable inorganic and organic acids. The salts obtained from pharmaceutically acceptable inorganic bases include ammonium, calcium, copper, ferric and ferrous salts, lithium, magnesium, manganic and manganous salts, potassium, sodium, zinc salts and the like. Especially preferred are the ammonium, calcium, magnesium, potassium and sodium salts. The salts obtained from pharmaceutically acceptable organic bases include primary, secondary and tertiary amine salts, including substituted amines, cyclic amines, natural amines and the like, such as arginine, betaine, caffeine, choline, N,N'-2-dibenzylethylendiamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like. The salts obtained from pharmaceutically acceptable acids include acetic, ascorbic, benzene sulphonic, benzoic, camphosulphonic, citric, ethanesulphonic, edisylic, fumaric, gentisic, gluconic, glucuronic, glutamic, hippuric, hydrobromic, hydrochloric, isethionic, lactic, lactobionic, maleic, malic, mandelic, methanesulphonic, mucic, naphthalenesulphonic, naphthalene-1,5-disulphonic, naphthalene-2,6-disulphonic, nicotinic, nitric, orotic, pamoic, pantothenic, phosphoric, succinic, sulphuric, tartaric, p-toluenesulphonic, xinafoic and the like. Particularly preferred are citric, hydrobromic, hydrochloric, isethionic, maleic, naphthalene-1,5-disulphonic, phosphoric, sulphuric and tartaric acids.

Table 1 shows the meaning of the substituents for each compound:

**Table 1**

| Example | R₁ | R₂ |
|---|---|---|
| 1 | tBu | Me |
| 2 | iPr | Me |
| 3 | Et | Me |
| 4 | Me | Me |
| 5 | cPr | Me |
| 6 | Bu | Me |
| 7 | iBu | Me |
| 8 | OMe | Me |
| 9 | OEt | Me |
| 10 | CF₃ | Me |
| 11 | MeCHF | Me |
| 12 | EtNH | Me |

Another aspect of the present invention is to provide a compound of the invention for use in the treatment or prevention of melatoninergic disorders. Said melatoninergic disorders are chosen from depression, stress, sleep disorders, anxiety, seasonal affective disorders, cardiovascular pathologies, digestive system pathologies, insomnia or fatigue due to jet lag, schizophrenia, panic attacks, melancholia, appetite disorders, obesity, insomnia, psychotic diseases, epilepsy, diabetes, Parkinson's disease, senile dementia, disorders associated to normal or pathological aging, migraine, memory loss, Alzheimer's disease and brain circulation disorders.

Another aspect of the present invention is to provide pharmaceutical compositions comprising a compound of the invention and one or more pharmaceutically acceptable excipients.

Another aspect of the present invention is to provide the use of said pharmaceutical compositions in the preparation of a medicinal product for the treatment or prevention of melatoninergic disorders. Said melatoninergic disorders are chosen from depression, stress, sleep disorders, anxiety, seasonal affective disorders, cardiovascular pathologies, digestive system pathologies, insomnia or fatigue due to jet lag, schizophrenia, panic attacks, melancholia, appetite disorders, obesity, insomnia, psychotic diseases, epilepsy, diabetes, Parkinson's disease, senile dementia, disorders associated to normal or pathological aging, migraine, memory loss, Alzheimer's disease and brain circulation disorders.

How to obtain compounds of general formula I is described in the following Diagram 1, where the substituents R₁ and R₂ have been described above, showing for R₂ = Me.

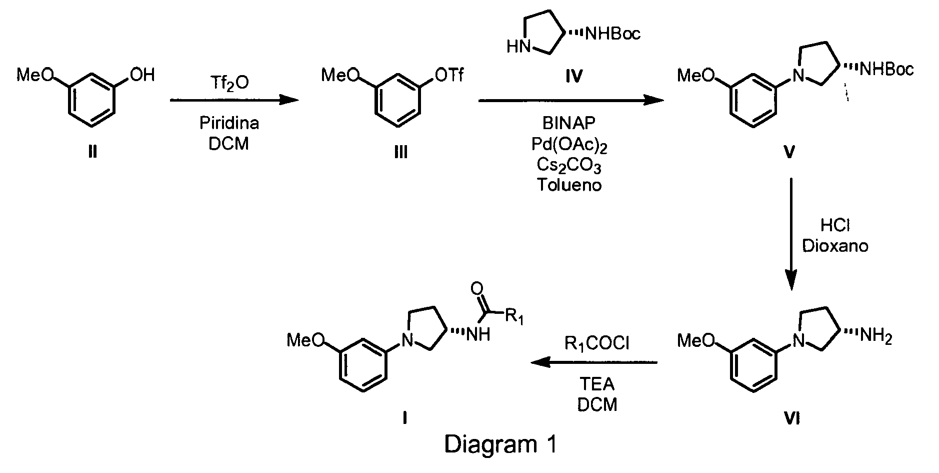

The first step consists in activating the hydroxyl group present in 3-methoxyphenol II. Said hydroxyl group is made to react with triflic anhydride in pyridine and dichloromethane to obtain the corresponding triflate III. The following step of the synthesis consists in a Buchwald reaction between the prior activated phenol III and the protected aminopyrrolidin IV, which is commercially available. Said reaction, provides phenylpyrrolidine V by triflate substitution. Deprotection of the Boc group present in V in an acid medium yields VI. Finally the last step consists in a usual coupling between amine VI and acid chlorides to yield compounds I. Similarly, when the final products I are ureas or carbamates, the coupling reagents are the appropriate isocyanates or chloroformiates, respectively.

Pharmaceutical compositions comprising compounds of the present invention include those that are adequate for oral, rectal and parenteral administration (including the subcutaneous, intramuscular and intravenous routes), although the most suitable route will depend on the nature and seriousness of the pathology being treated. The preferred administration route for the compounds of the present invention is frequently the oral route.

The active ingredients can be mixed with one or more pharmaceutical excipients following conventional pharmaceutical techniques for formulation. Several excipients can be used according to the pharmaceutical form to be prepared. Liquid oral compositions (such as, for example, suspensions, solutions, emulsions, aerosols and mouthwashes) may use, for example, water, glycols, oils, alcohols, flavour enhancers, preservatives, colorants and the like. Solid oral compositions use, for example, starches, sugars (such as, for example, lactose, sucrose and sorbitol), celluloses (such as, for example, hydroxypropyl cellulose, carboxymethyl cellulose, ethyl cellulose and microcrystalline cellulose), talc, stearic acid, magnesium stearate, dicalcium phosphate, rubbers, copovidone, surfactants such as sorbitan monooleate and polyethyleneglycol, metallic oxides (such as, for example, titanium dioxide and ferric oxide) and other pharmaceutical diluents such as water. Homogeneous preformulations are thus formed containing the compounds of the present invention.

In the case of the preformulations the compositions are homogeneous, such that the active ingredient is dispersed uniformly in the composition, which can therefore be divided in equal unit doses such as tablets, coated tablets, powders and capsules.

Tablets and capsules are most advantageous oral forms due to their ease of administration. Tablets can be coated using aqueous or nonaqueous conventional techniques if so desired. A large variety of materials can be used to form the coating. Such materials include a large number of polymeric acids and their mixtures with other components such as, for example, shellac, cetyl alcohol and cellulose acetate.

Liquid forms in which the compounds of the present invention can be incorporated for oral or injectable administration include aqueous solutions, capsules filled with fluid or gel, syrups with flavour enhancers, aqueous suspensions in oil and emulsions flavoured with edible oils such as, for example, cottonseed oil, sesame oil, coconut oil or peanut oil, as well as mouthwashes and similar pharmaceutical carriers. Suitable dispersing or suspension agents for the preparation of aqueous suspensions include synthetic and natural gums such as tragacanth, Acacia, alginates, dextranes, sodium carboxymethylcellulose, methylcellulose, polyethyleneglycol, polyvinylpyrrodidone or gelatin.

A suitable dosage range to be used is a total daily dose from 0.1 to 500 mg approximately, more preferably from 1 mg to 100 mg, either in a single administration or in separate doses if necessary.

### Embodiments of the invention

The present invention is additionally illustrated by means of the following examples, which do not intent to limit the scope thereof.

### Example of pharmacological assessment 1

### Determination of the agonist activity on MT1 receptors

In order to screen compounds for the MT1 receptor a cell line is used that is characterised by stable overexpression of the recombinant human MT1 receptor in a cell line that in turn co-expresses mitochondrial apoaequorin and the Gα16 subunit.

The Gα16 subunit belongs to the G protein family, formed by GPCR, wherein the transduction of intracellular signals occurs via phospholipase (PLC). PLC activation produces an increase in inositol-triphosphate levels that leads to an increase in intracellular calcium. Gα16 overexpression thus allows an increase in intracellular calcium levels that is independent and compatible with the study receptor's own signal transduction pathway.

Apoaequorin is the inactive form of aequorin, a phosphoprotein that requires a hydrophobic prosthetic group, coelenterazine, to produce the active form. Following its binding to calcium, the aequorin oxidises coelenterazine to coelenteramide, a reaction that releases CO₂ and light.

The trial protocol for the screening of possible agonists consists in collecting the cells and keeping them in suspension overnight in the presence of coelenterazine in order to reconstitute aequorin. On the following day the cells are injected on a plate where the compounds to be screened are diluted, and the luminescence released is read immediately. When wishing to study the possible antagonism of the same compounds, the reference agonist compound is added in the same well after 15-30 min from the first injection and the luminescence released is assessed.

Agonist activity is calculated as percentage activity with respect to the reference agonist at the concentration corresponding to its EC100. Antagonist activity is expressed as percentage inhibition over the reference agonist activity at the concentration corresponding to its EC80.

### Example of pharmacological assessment 2

### Determination of agonist activity on MT2 receptors

In order to study agonism against MT2 receptors we use a recombinant cell line that expresses these receptors and coexpresses mitochondrial apoaequorin and the Gα16 subunit, as in the model used for MT1 screening. The compounds of the present invention show in this model that they also have agonism for the MT2 receptors.

Table 2 shows the results for agonism on the MT1 receptors versus the ramelteon, melatonin and (1S)-N-[2-(6-methoxy-indan-1-yl)-ethyl]-propionamide standards (WO 9608466 and O. Uchikawa et al., J. Med. Chem., 2002, 45, 4222-4239; compound 60), demonstrating that the compounds of the present invention exhibit comparable activity to that of said reference compounds.

**Table 2**

| Compound | MT1 agonism | |
|---|---|---|
| | 100 nM | 1 nM |
| Example 3 | 109.9 | 46.7 |
| Example 4 | 102.7 | 34.4 |
| Example 6 | 99.6 | 33.4 |
| Example 7 | 110.3 | 50.1 |
| Ramelteon | 117.5 | 50.0 |
| Melatonin | 100.5 | 51.8 |
| (1S)-N-[2-(6-Methoxy-indan-1-yl)-ethyl]-propionamide | 102.7 | 37.1 |

Moreover, the compounds of the present invention advantageously provide relevant pharmacokinetic improvements. Therefore, studies of metabolic stability determined by the disappearance of the compounds to be tested by incubation in human microsomes for 120 min at 1 µM and studies for the determination of rat plasma levels (ng/mL) 15 min after the administration of 1 mg/Kg of the compounds to be tested have shown that the compound from example 8 has high metabolic stability (comprised between 71% and 100%), plasma levels of 15.1 ng/mL and a brain/plasma ratio of 1, insofar as comparatively (1S)-N-[2-(6-methoxy-indan-1-yl)-ethyl]-propionamide shows low metabolic stability (less than 30%), plasma levels of 10.1 ng/mL and a brain/plasma ratio close to zero. Consequently, the compound from example 8, despite certain structural similarities with the reference compound, shows unexpectedly higher pharmacokinetic properties as a result of its greater metabolic stability, greater plasma levels and a greater brain/plasma ratio.

In short, the present invention provides new compounds that, despite having certain structural similarity with compounds of the state of the art, surprisingly show lower biotransformation and higher levels both in the brain and in plasma, thus providing more sustained sleep.

### Reference example 1

### General procedure for obtaining triflates III

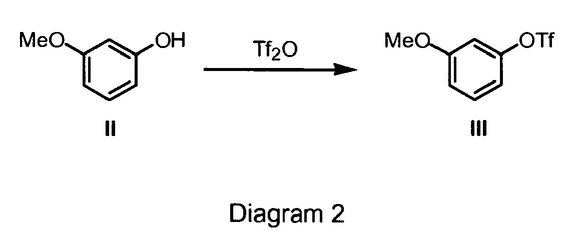

Pyridine (28.7 mL, 354 mmol) is added to a solution of 3-methoxyphenol II (17.68 mL, 161 mmol) in dichloromethane (DCM) (250 mL). It is cooled in a water-ice bath. Trifluoromethansulphonic anhydride (Tf2O, 29.8 mL, 177 mmol) is slowly added for 30 min. During this addition the temperature is kept at all times below 10°C. It is stirred at 0°C for 30 min, and it is allowed to reach ambient temperature for 3 h 30 min. The solution is collected and washed with a solution saturated of metabisulphite and water. The organic phase is dried over anhydrous sodium sulphate, filtered, and the solvent is evaporated under low pressure. 45 g of an oil III are obtained that are used directly in the following step of the synthesis.

### Reference example 2

### General procedure for obtaining compounds V

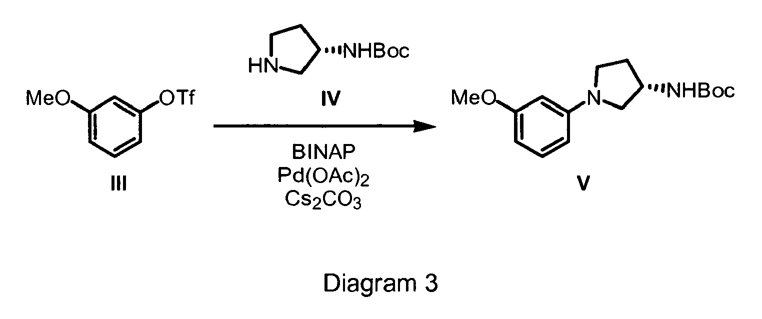

100 mL of toluene are taken and degassed by strong Ar bubbling for 10 min. 2.51 g (4.03 mmol) of [1,1'-binaphthalene]-2,2'-diylbis[diphenylphosphino] (BINAP) and 0.61 g (2.68 mmol) of palladium acetate are added. It is stirred for 15 min at room temperature. 14.52 g (44.6 mmol) of caesium carbonate and 5 g (26.8 mmol) of aminopyrrolidin IV are added. Finally, 5.78 g (22.5 mmol) of the triflate III are added in 10 mL of toluene. It is heated under reflux for 16 h. Allow to cool and filter the reaction crude. The solid is washed with DCM/water. The organic phase is taken, dried and filtered. A residue is obtained that is purified by column chromatography using ethyl acetate/hexane as an eluant mixture. 3.3 g (Yield = 42%) of the protected amine V are obtained as a yellow oil.

HPLC-MS: Purity 100%, M+1= 293

### Reference example 3

### General procedure for obtaining amines VI

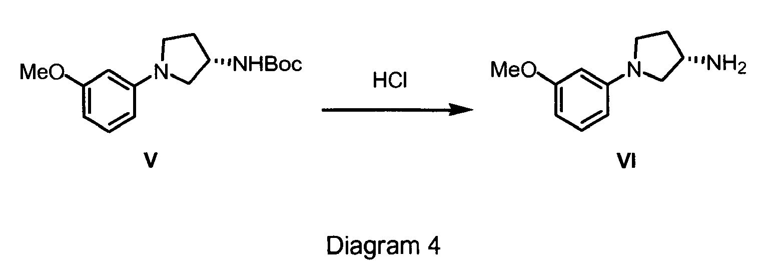

3.3 g (11.29 mmol) of the protected amine V are taken and 56.4 mL (226 mmol) of a solution of 4N HCl in dioxane are added. A new solid is seen to appear as the starting product dissolves. Stirring is continued for 1 h at room temperature. The dioxane is evaporated under low pressure and the solid obtained is suspended in DCM. It is washed with 3 portions of 50 mL of 3N NaOH. The organic phase is separated and dried over anhydrous magnesium sulphate. The solvent was filtered and evaporated. 2.09 g (Yield = 96%) of VI are obtained as a colourless oil.

HPLC-MS: Purity 98%, M+1= 193

### Reference example 4

### General procedure for obtaining compounds I

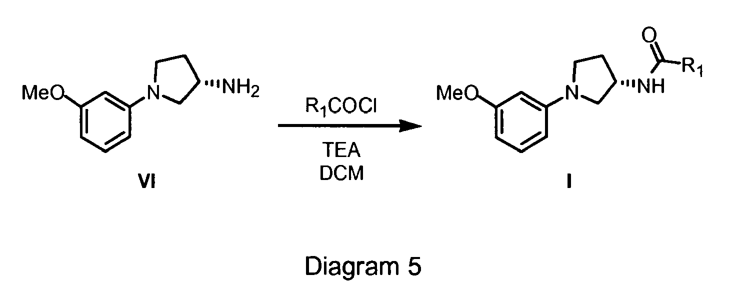

300 mg of amine V (1.56 mmol) are dissolved in 15 mL of anhydrous DCM. 0.395 mL of TEA (triethylamine) (2.84 mmol) are slowly added and subsequently 1.42 mmol of the corresponding acid chloride are also slowly added. Stir at room temperature for 1 h and 30 min. 10 mL of 1N HCl area added and it is stirred for 15 min. Separate the organic phase and dry. It is evaporated to dryness. The residue thus obtained is purified by column chromatography using ethyl acetate/hexane as eluants. The type I compounds are thus obtained as a white solid.

Example when R₁ = Me: 199.8 mg (Yield = 60%) are obtained.
HPLC-MS: Purity 98%, M+1= 235

The compounds thus obtained are detailed in the following Table 3.

**Table 3**

| Example | R₁ | R₂ | LCMS Purity (%) | M+1 |
|---|---|---|---|---|
| 1 | tBu | Me | 98 | 277 |
| 2 | iPr | Me | 97 | 263 |
| 3 | Et | Me | 96 | 249 |
| 4 | Me | Me | 98 | 235 |
| 5 | cPr | Me | 97 | 261 |
| 6 | Bu | Me | 99 | 277 |
| 7 | iBu | Me | 97 | 277 |
| 8 | OMe | Me | 100 | 251 |
| 9 | OEt | Me | 97 | 265 |
| 10 | CF₃ | Me | 98 | 289 |
| 11 | MeCHF | Me | 100 | 267 |
| 12 | EtNH | Me | 99 | 264 |

## Claims

1. Phenylpyrrolidine compounds selected from the group consisting of:
1) (S)-N-[1-(3-methoxy-phenyl)-pyrrolidin-3-yl]-2,2-dimethyl-propionamide;
2) (S)-N-[1-(3-methoxy-phenyl)-pyrrolidin-3-yl]-isobutyramide;
3) (S)-N-[1-(3-methoxy-phenyt)-pyrrolidin-3-yl]-propionamide;
4) (S)-N-[1-(3-methoxy-phenyl)-pyrrolidin-3-yl]-acetamide;
5) (S)-N-[1-(3-methoxy-phenyl)-pyrrolidin-3-yl]-cyclopropanecarboxamide;
6) (S)-N-[1-(3-methoxy-phenyl)-pyrrolidin-3-yl]-pentanamide;
7) (S)-N-[1-(3-methoxy-phenyl)-pyrrolidin-3-yl]-3-methyl-butyramide;
8) Methyl (S)-[1-(3-methoxy-phenyl)-pyrrolidin-3-yl]-carbamate;
9) Ethyl (S)-[1-(3-methoxy-phenyl)-pyrrolidin-3-yl]-carbamate;
10) (S)-2,2,2-trifluoro,N-[1-(3-methoxy-phenyl),pyrrolidin-3-yl]-acetamide;
11) (S)-2-fluoro-N-[1-(3-methoxy-phenyl)-pyrrolidin-3-yl]-propionamide; and
12) (S)-3-[1-(3-methoxy-phenyl)-pyrrolidin-3-yl]-1-ethylurea;
and pharmaceutically acceptable salts and hydrates thereof.

2. A compound of claim 1 for use in the treatment or prevention of melatoninergic disorders.

3. The compound of claim 2 wherein said melatoninergic disorders are chosen from depression, stress, sleep disorders, anxiety, seasonal affective disorders, cardiovascular pathologies, digestive system pathologies, insomnia or fatigue due to jet lag, schizophrenia, panic attacks, melancholia, appetite disorders, obesity, insomnia, psychotic diseases, epilepsy, diabetes, Parkinson's disease, senile dementia, disorders associated to normal or pathological aging, migraine, memory loss, Alzheimer's disease and brain circulation disorders.

4. A pharmaceutical composition comprising a compound of claim 1 and one or more pharmaceutically acceptable excipients.

5. The pharmaceutical composition of claim 4 for use in the treatment or prevention of melatoninergic disorders.

6. The composition of claim 5 wherein said melatoninergic disorders are chosen from depression, stress, sleep disorders, anxiety, seasonal affective disorders, cardiovascular pathologies, digestive system pathologies, insomnia or fatigue due to jet lag, schizophrenia, panic attacks, melancholia, appetite disorders, obesity, insomnia, psychotic diseases, epilepsy, diabetes, Parkinson's disease, senile dementia, disorders associated to normal or pathological aging, migraine, memory loss, Alzheimer's disease and brain circulation disorders.

## Patentansprüche

1. Phenylpyrrolidin-Verbindungen, ausgewählt aus der Gruppe, bestehend aus:
1) (S)-N-[1-(3-Methoxy-phenyl)-pyrrolidin-3-yl]-2,2-dimethyl-propionamid;
2) (S)-N-[1-(3-Methoxy-phenyl)-pyrrolidin-3-yl]-isobutyramid;
3) (S)-N-[1-(3-Methoxy-phenyl)-pyrrolidin-3-yl]-propionamid;
4) (S)-N-[1-(3-Methoxy-phenyl)-pyrrolidin-3-yl]-acetamid;
5) (S)-N-[1-(3-Methoxy-phenyl)-pyrrolidin-3-yl]-cyclopropancarboxamid;
6) (S)-N-[1-(3-Methoxy-phenyl)-pyrrolidin-3-yl]-pentanamid;
7) (S)-N-[1-(3-Methoxy-phenyl)-pyrrolidin-3-yl]-3-methyl-butyramid;
8) Methyl-(S)-[1-(3-methoxy-phenyl)-pyrrolidin-3-yl]-carbamat;
9) Ethyl-(S)-[1-(3-methoxy-phenyl)-pyrrolidin-3-yl]-carbamat;
10) (S)-2,2,2-Trifluor-N-[1-(3-methoxy-phenyl)-pyrrolidin-3-yl]-acetamid;
11) (S)-2-Fluor-N-[1-(3-methoxy-phenyl)-pyrrolidin-3-yl]-propionamid; und
12) (S)-3-[1-(3-Methoxy-phenyl)-pyrrolidin-3-yl]-1-ethylharnstoff;
und pharmazeutisch akzeptable Salze und Hydrate davon.

2. Verbindung nach Anspruch 1 zur Verwendung in der Behandlung oder Prävention von melatonergen Störungen.

3. Verbindung nach Anspruch 2, wobei die melatonergen Störungen ausgewählt sind unter Depression, Stress, Schlafstörungen, Angst, jahreszeitlich bedingten Depressionen, kardiovaskulären Pathologien, Pathologien des Verdauungsapparates, durch Jetlag verursachter Schlaflosigkeit oder Müdigkeit, Schizophrenie, Panikattacken, Melancholie, Appetitstörungen, Fettsucht, Schlaflosigkeit, psychotischen Erkrankungen, Epilepsie, Diabetes, Morbus Parkinson, Altersdemenz, Störungen in Verbindung mit normalem oder pathologischem Altern, Migräne, Gedächtnisverlust, Alzheimer-Krankheit und Durchblutungsstörungen des Gehirns.

4. Pharmazeutische Zusammensetzung, umfassend eine Verbindung nach Anspruch 1 und einen oder mehrere pharmazeutisch akzeptable Exzipienten.

5. Pharmazeutische Zusammensetzung nach Anspruch 4 zur Verwendung in der Behandlung oder Prävention von melatonergen Störungen.

6. Zusammensetzung nach Anspruch 5, wobei die melatonergen Störungen ausgewählt sind unter Depression, Stress, Schlafstörungen, Angst, jahreszeitlich bedingten Depressionen, kardiovaskulären Pathologien, Pathologien des Verdauungsapparates, durch Jetlag verursachter Schlaflosigkeit oder Müdigkeit, Schizophrenie, Panikattacken, Melancholie, Appetitstörungen, Fettsucht, Schlaflosigkeit, psychotischen Erkrankungen, Epilepsie, Diabetes, Morbus Parkinson, Altersdemenz, Störungen in Verbindung mit normalem oder pathologischem Altern, Migräne, Gedächtnisverlust, Alzheimer-Krankheit und Durchblutungsstörungen des Gehirns.

## Revendications

1. Composés Phénylpyrrolidiniques sélectionnés dans le groupe composé de :
1) (S)-N-[1-(3-méthoxy-phényl)-pyrrolidine-3-yl]-2,2-diméthyl-propionamide ;
2)(S)-N-[1-(3-méthoxy-phényl)-pyrrolidine-3-yl]-isobutyramide ;
3)(S)-N-[1-(3-méthoxy-phényl)-pyrrolidine-3-yl]-propionamide ;
4)(S)-N-[1-(3-méthoxy-phényl)-pyrrolidine-3-yl]-acétamide ;
5)(S)-N-[1-(3-méthoxy-phényl)-pyrrolidine-3-yl]-cyclopropanecarboxamide ;
6)(S)-N-[1-(3-méthoxy-phényl)-pyrrolidine-3-yl]-pentanamide ;
7)(S)-N-[1-(3-méthoxy-phényl)-pyrrolidine-3-yl]-3-méthyl-butyramide ;
8)(S)-[1-(3-méthoxy-phényl)-pyrrolidine-3-yl]-carbamate de méthyle ;
9)(S)-[1-(3-méthoxy-phényl)-pyrrolidine-3-yl]-carbamate d'éthyle ;
10)(S)-2,2,2-trifluoro-N-[1-(3-méthoxy-phényl)-pyrrolidine-3-yl]-acétamide ;
11) (S)-2-fluoro-N-[1-(3-méthoxy-phényl)-pyrrolidine-3-yl]-propionamide ; et
12) (S)-3-[1-(3-méthoxy-phényl)-pyrrolidine-3-yl]-1-éthylurée ;
et leurs sels et hydrates pharmaceutiquement acceptables.

2. Composé de la revendication 1 à utiliser dans le traitement ou la prévention de troubles mélatoninergiques.

3. Composé de la revendication 2 dans lequel lesdits troubles mélatoninergiques sont choisis parmi la dépression, le stress, les troubles du sommeil, l'anxiété, les troubles affectifs saisonniers, les pathologies cardiovasculaires, les pathologies du système digestif, l'insomnie ou la fatigue due au décalage horaire, la schizophrénie, les attaques de panique, la mélancolie, les troubles de l'alimentation, l'obésité, l'insomnie, les maladies psychotiques, l'épilepsie, le diabète, la maladie de Parkinson, la démence sénile, des troubles associés au vieillissement normal ou pathologique, la migraine, la perte de mémoire, la maladie d'Alzheimer et les troubles de la circulation cérébrale.

4. Composition pharmaceutique comprenant un composé de la revendication 1 et un ou plusieurs excipients pharmaceutiquement acceptables.

5. Composition pharmaceutique de la revendication 4 à utiliser dans le traitement ou la prévention de troubles mélatoninergiques.

6. Composition de la revendication 5, dans laquelle lesdits troubles mélatoninergiques sont choisis parmi la dépression, le stress, les troubles du sommeil, l'anxiété, les troubles affectifs saisonniers, les pathologies cardiovasculaires, les pathologies du système digestif, l'insomnie ou la fatigue due au décalage horaire, la schizophrénie, les attaques de panique, la mélancolie, les troubles de l'alimentation, l'obésité, l'insomnie, les maladies psychotiques, l'épilepsie, le diabète, la maladie de Parkinson, la démence sénile, des troubles associés au vieillissement normal ou pathologique, la migraine, la perte de mémoire, la maladie d'Alzheimer et les troubles de la circulation cérébrale.
